# EUROPEAN PATENT SPECIFICATION

(11) **EP 4 129 359 B1**
(45) Date of publication and mention of the grant of the patent: **20.12.2023**
(21) Application number: 22189034.6
(22) Date of filing: 05.08.2022
(51) Int. Cl.: A61M 1/36, A61M 1/38

(54) **SYSTEMS AND METHODS FOR SEPARATION OF PLATELETS FROM BLOOD AND RETURN OF MONONUCLEAR CELLS**
SYSTEME UND VERFAHREN ZUM ABTRENNEN VON BLUTPLÄTTCHEN UND ZUM RÜCKFÜHREN VON MONONUKLEÄREN ZELLEN
SYSTÈMES ET PROCÉDÉS DE SÉPARATION DE PLAQUETTES DU SANG ET DE RETOUR DE CELLULES MONONUCLÉAIRES

(30) Priority: 06.08.2021 US 202163230228 P
(43) Date of publication of application: 08.02.2023
(73) Proprietor: Fenwal, Inc., Lake Zurich, IL 60047 (US)
(72) Inventor: MIN, Kyungyoon, Lake Zurich, 60047 (US)
(74) Representative: Maikowski & Ninnemann Patentanwälte Partnerschaft mbB

(56) References cited:
- US-A- 5 868 696
- US-A- 6 027 657
- US-A1- 2012 316 051

## Description

### Field of the Disclosure

The invention relates to blood separation. More particularly, the invention relates to systems and methods for separating platelets from blood during a separation procedure and returning mononuclear cells to the blood source.

### Description of Related Art

Various blood processing systems make it possible to separate blood into two or more constituent parts, which may be useful for donation purposes and for treatment of individuals with potentially detrimental or harmful blood conditions or disorders.

When such systems are used for blood component donation, whole blood is typically drawn from a donor, the particular blood component or constituent is removed and collected, and the remaining blood constituents are returned to the donor. By thus removing only particular constituents, potentially less time is needed for the donor's body to return to normal, and donations can be made at more frequent intervals than when whole blood is collected. This increases the overall supply of blood constituents, such as plasma and platelets, made available for health care.

Whole blood is typically separated into its constituents through centrifugation. This requires that the whole blood be passed through a centrifuge after it is withdrawn from, and before it is returned to, the source. To avoid contamination of the blood and possible infection of the source (if the source is a living donor or patient), the blood is preferably contained within a sealed, sterile fluid flow system during the entire centrifugation process. Typical blood processing systems thus include a permanent, reusable centrifuge assembly containing the hardware (drive system, pumps, valve actuators, programmable controller, and the like) that spins and pumps the blood, and a disposable, sealed and sterile fluid processing assembly that is mounted in cooperation on the hardware. The centrifuge assembly engages and spins a disposable separation chamber of the fluid processing assembly during a collection or treatment procedure. The blood, however, makes actual contact only with the fluid processing assembly, which assembly is used only once and then discarded.

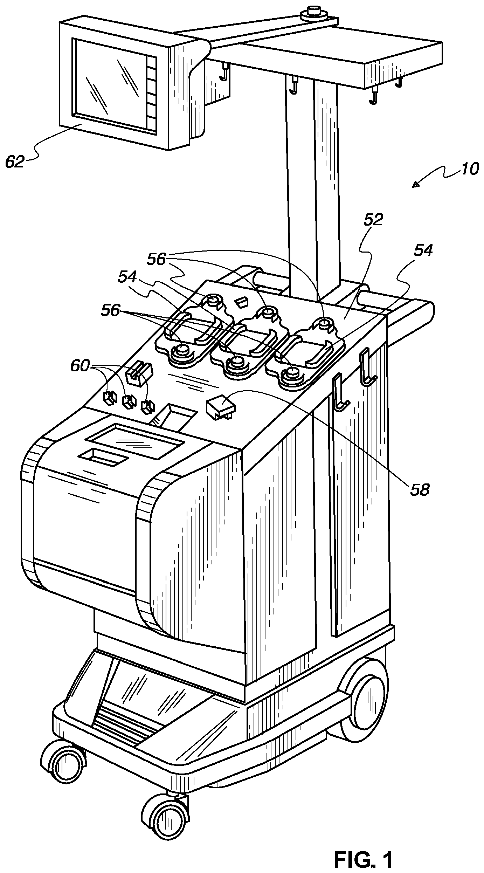

Centrifugal blood separation devices are known in the art and currently practiced commercially. One known separation device is shown in Figs. 1-7.

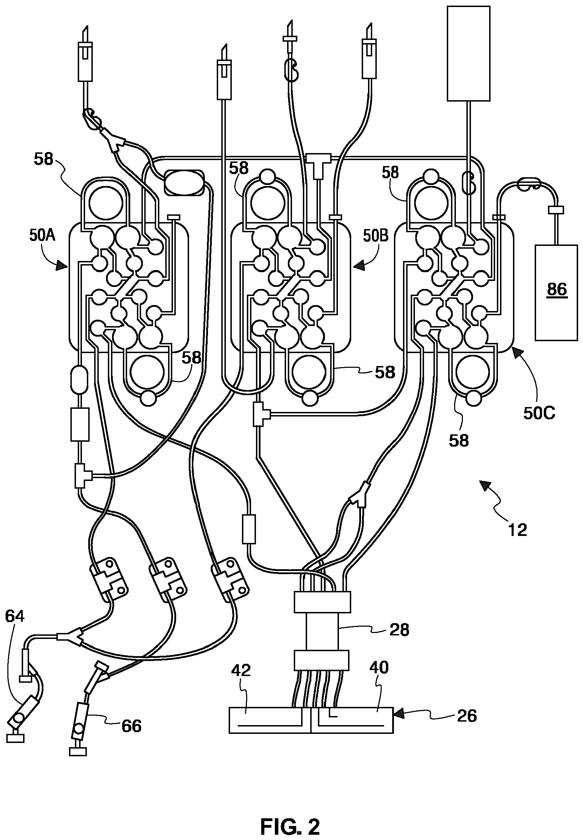

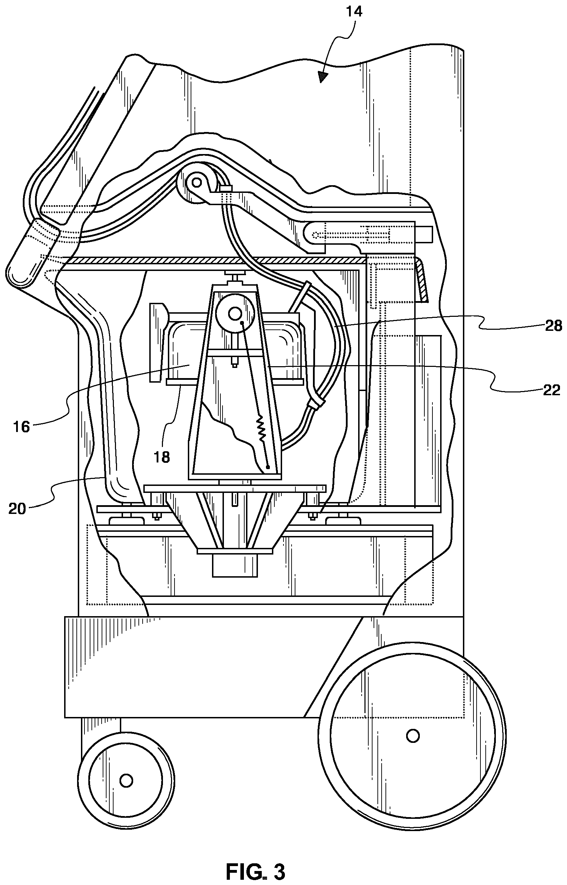

Fig. 1 shows an exemplary commercial centrifugal blood separation device 10 that may be used in combination with a disposable fluid flow circuit 12 (Fig. 2) to comprise a blood processing system (Fig. 3) for separating blood into two or more components. The illustrated blood separation device 10 is currently marketed as the AMICUS^{®} separator by Fenwal, Inc. of Lake Zurich, Illinois, which is an affiliate of Fresenius Kabi AG of Bad Homburg, Germany, and is described in greater detail in U.S. Patent No. 5,868,696. The device 10 can be used for processing various fluids, but is particularly well suited for processing whole blood, blood components, or other suspensions of biological cellular materials.

The device 10 includes a separator configured as a centrifuge 14 (Fig. 3) used to centrifugally separate blood components. The device 10 may be programmed to separate blood into a variety of components and sub-components. For example, in an exemplary blood separation procedure, the centrifuge 14 is operated to separate whole blood into platelet-rich plasma and red blood cells, with the platelet-rich plasma then being separated into platelet-poor plasma and platelets or platelet concentrate.

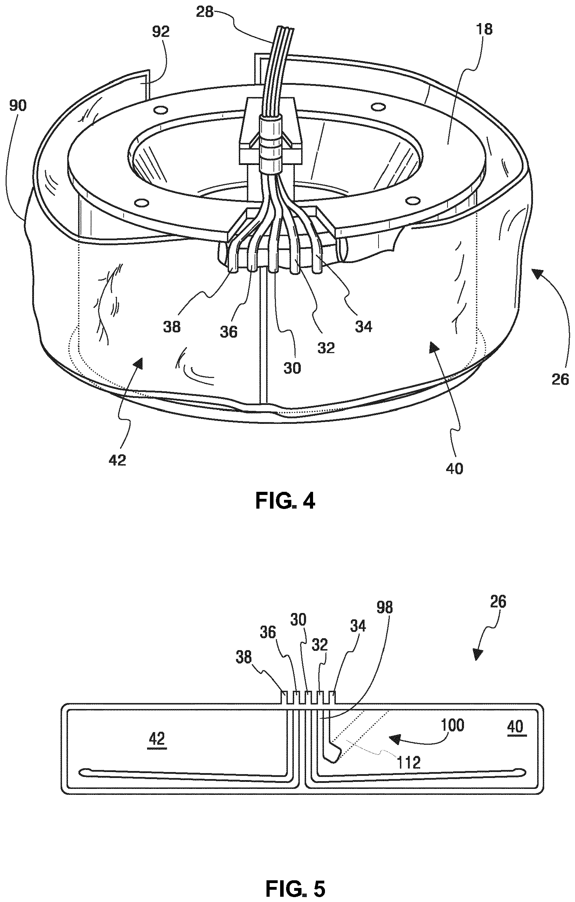

The illustrated centrifuge 14 is of the type shown in U.S. Patent No. 5,316,667. The centrifuge comprises a bowl 16 and a spool 18, which are received within a bucket or enclosure 20. The bowl 16 and spool 18 are pivoted on a yoke 22 between an operating position and a loading/unloading position. When the bowl 16 and spool 18 are in the loading/unloading position, a separation chamber 26 of the flow circuit 12 is wrapped around the spool 18 (Fig. 4) and positioned within the centrifuge 14, in an annular gap defined between the bowl 16 and the spool 18. Further details of a separation chamber 26 of the type shown in Fig. 4 and its operation may also be found in U.S. Patent No. 5,316,667.

The chamber 26 is typically formed of a pair of flexible, deformable sheets of material (e.g., a polyvinyl chloride material) that are sealed together at their perimeter. One of the sheets defines a high-G (outer) wall 90 when the chamber 26 is mounted into the centrifuge 14, while the other sheet defines a low-G (inner) wall 92 that is positioned closer to the rotational axis of the centrifuge 14. A plurality of ports 30, 32, 34, 36, and 38 extend through the sealed perimeter to allow fluid flow between the interior of the chamber 26 and the other components of the flow circuit 12.

The illustrated chamber 26 further includes a plurality of interior seals that define first and second chambers 40 and 42 and direct the flow of separated blood components within the chamber 26. As shown in Fig. 5, there are three ports 30, 32, and 34 associated with the first stage 40 of the illustrated chamber 26. The port identified at 32 is used for conveying blood from a blood source into the first stage 40. The other two ports 30 and 34 serve as outlet ports for separated blood components exiting the first stage 40. More particularly, the first outlet port 34 conveys a low density blood component from the first stage 40, while the second outlet port 30 conveys a high density blood component from the first stage 40.

Other portions of the flow circuit 12 may remain outside of the bucket 20. In the illustrated embodiment, the various tubing connected to the blood separation chamber 26 are bundled in an umbilicus 28, which extends outside of the bucket 20 during use (Fig. 3). As shown in Figs. 4 and 5, the umbilicus 28 of the flow circuit 12 may be attached to ports 30, 32, 34, 36, and 38 of the blood separation chamber 26 by individual tubing. The umbilicus 28 interconnects first and second stages 40 and 42 of the chamber 26 with each other and with the components of the flow circuit 12 positioned outside of the centrifuge 14 during use.

The tubing of the umbilicus 28 may be connected to cassettes 50A, 50B, and 50C of the flow circuit 12 (Fig. 2), which are molded components that define a plurality of fluid flow segments that may be selectively placed into and out of fluid communication with each other via the operation of valve stations defined in the cassette. A front panel 52 of the blood separation device 10 includes a plurality of cassette holders 54 (Fig. 1) to accommodate the cassettes 50A, 50B, and 50C of the flow circuit 12. Each cassette holder 54 receives and grips a different one of the cassettes 50A, 50B, 50C of the fluid flow circuit 12 along two opposed side edges in the desired operating position. Each cassette holder 54 includes a pair of peristaltic pump stations or pumps 56. When a cassette is gripped by the cassette holder 54, tubing loops 58 extending from the cassette (Fig. 2) make operative engagement with the pumps 56. The pumps 56 are operated under command of a system controller to cause fluid flow through the associated cassette.

The front panel 52 of the device 10 may include additional components, such as at least one optical line monitor 58. If provided, the optical line monitor 58 may receive a tubing or fluid flow conduit of the flow circuit 12 to optically monitor fluid flowing therethrough. The front panel 52 may also include various clamps 60 that receive a tubing or fluid flow conduit of the flow circuit 12 to selectively allow and prevent fluid flow through that conduit.

A user interface screen 62 (e.g., a touchscreen) may be positioned above the front panel 52 (as in Fig. 1) or at some other location. The user interface screen 62 may allow an operator to interact with the system controller (e.g., a microprocessor) of the device 10 to provide instructions to the controller (e.g., to carry out a particular procedure), as well as providing information to the controller to be used during a procedure (e.g., a platelet pre-count of the blood of the blood source). The user interface screen 62 may provide the operator with instructions (e.g., to connect or disconnect the blood source from the flow circuit 12) and information (e.g., alerting the operator to a blockage in a fluid flow conduit of the flow circuit 12).

As noted, the various components of the fluid flow circuit 12 may be connected by flexible tubing or any other suitable fluid flow conduit. The illustrated flow circuit 12 is a "two needle" system, which includes a pair of blood source access devices 64 and 66 (e.g., phlebotomy needles), with one serving to draw blood into the flow circuit 12 from a source, while the other serves to return fluid to the source. In other embodiments, the flow circuit may be configured as a "single needle" system in which a single blood source access device (e.g., a phlebotomy needle) is used to both draw blood from a blood source and convey fluid to the blood source.

To begin a separation procedure, an operator may select (e.g., using the user interface screen 62) the procedure from among the variety of procedures that the device 10 is capable of performing. The operator may enter a variety of information requested by the system controller that allows the controller to better carry out the procedure. The controller can be provided with the total blood volume of the blood source, a platelet pre-count or the initial platelet concentration of the blood of the blood source, and a platelet post-count or a target platelet concentration to be achieved for the blood of the blood source by the end of the procedure. The total amount of blood to be processed may also be provided to the system controller.

When the system controller has received all of the necessary input, performed the necessary preliminary calculations and status checks (e.g., to confirm that the flow circuit 12 is properly installed and that the various components of the device 10 are functioning properly), and primed the flow circuit 12, the separation procedure may begin.

The system controller instructs one or more of the pumps 56 to draw blood from the blood source into the flow circuit 12 via one of the blood source access devices 64. The blood enters the first stage 40 of the separation chamber 26 via the inlet port 32 as the centrifuge 14 rotates the chamber 26 about a rotational axis at a sufficient speed so that the blood is separated into red blood cells (i.e., the high density blood component) and platelet-rich plasma (i.e., the low density blood component). The red blood cells are returned to the blood source via the outlet port 30, while the platelet-rich plasma is conveyed out of the first stage 40 via the outlet port 34 and into the second stage 42 via the inlet port 38. In the second stage 42, the platelet-rich plasma is separated into platelet-poor plasma and platelets or platelet concentrate. The platelet-poor plasma is removed from the second stage 42 and may be returned to the blood source via the outlet port 36, leaving the platelets/platelet concentrate to accumulate in the second stage 42 for eventual transfer to a collection container 86.

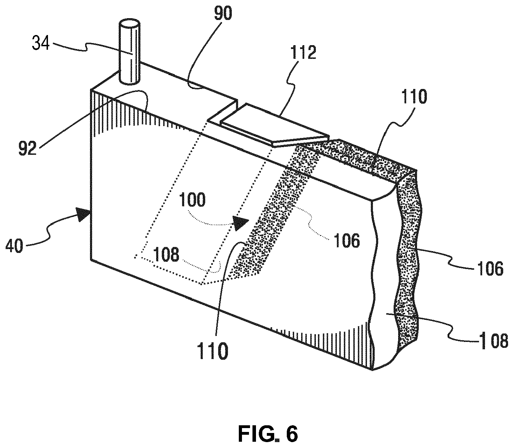

More particularly, during the separation procedure, a fluid passage 98 associated with the inlet port 32 channels blood directly into the circumferential flow path immediately next to a low density collection region 100. As shown in Fig. 6, the blood separates into an optically dense layer 106, which forms as cellular components move under the influence of centrifugal force toward the high-G (outer) wall 90 of the chamber 26. The optically dense layer 106 will include red blood cells (and, hence, will be referred to herein as the "RBC layer") but, depending on the speed at which the centrifuge 14 is spun, other cellular components (e.g., larger white blood cells and platelets) may also be present in the RBC layer 106.

The movement of the component(s) of the RBC layer 106 displaces less dense blood components radially toward the low-G (inner) wall 92 of the chamber 26, forming a second, less optically dense layer 108. The less optically dense layer 108 includes plasma and platelets (and, hence, will be referred to herein as the "PRP layer") but, depending on the speed at which the centrifuge 14 is rotated and the length of time that the blood is resident in the centrifuge 14, other components (e.g., smaller white blood cells) may also be present in the PRP layer 108.

The transition between the RBC and PRP layers 106 and 108 is generally referred to as the interface 110 (Fig. 6). The location of the interface 110 within the chamber 26 can dynamically shift during blood processing. If the location of the interface 110 is too high (that is, if it is too close to the low-G wall 92 and the outlet port 34), red blood cells can spill over and into the low density collection region 100, adversely affecting the quality of the platelet-rich plasma. On the other hand, if the location of the interface 110 is too low (that is, if it resides too far away from the low-G wall 92), the collection efficiency of the device 10 may be impaired. The blood separation device 10 may include a viewing head or interface sensor assembly to optically view and adjust the position of the interface 110, which is visible on an interface ramp 112.

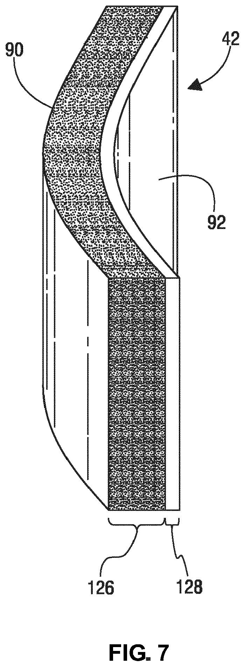

While the blood is being separated into the RBC layer 106 and the PRP layer 108 in the first stage 40, the portion of the PRP layer 108 in the second stage 42 (received from the first stage 40) is separating into platelets or platelet concentrate 126 and platelet-poor plasma 128 (Fig. 7), as described above. At the same time, a buffy coat comprising (among other things) mononuclear cells (referred to herein as "MNCs") and larger platelets develops within the first stage at or adjacent to the interface 110. As noted above, the interface 110 is monitored to ensure that it remains at a suitable position within the first stage 40, which causes the buffy coat to remain within the first stage 40 during blood separation (rather than exiting the first stage 40 with either the RBC layer 106 or the PRP layer 108), increasing in volume.

Blood draw and separation continue until they are ended by the system controller. The platelets/platelet concentrate 126 accumulating in the second stage 42 are then harvested or collected, followed by a reinfusion stage in which the fluid that remains in the chamber 26 (including the buffy coat) is returned to the blood source.

While platelet separation and collection methods of this type have proven to be effective, it has been found recently that they may lead to a donor or patient experiencing leukopenia, due to insufficient return of the mononuclear cell components in the buffy coat. For example, it has been found that procedures of the type described above may leave on the order of 1 x 10⁸ MNCs in the separation chamber, rather than the MNCs being returned to the blood source.

This problem may be even greater in systems omitting a post-collection reinfusion stage of the type described above. For example, it has been found that platelet collection or depletion procedures carried out using the TRIMA ACCEL^{®} collection device marketed by Terumo BCT, Inc. of Lakewood, Colorado may fail to return on the order of 1 x 10⁹ - 1 x 10¹⁰ MNCs to a patient or donor.

A procedure leaving behind fewer MNCs as waste (e.g., on the order of 1 x 10⁷ MNCs) would greatly decrease the occurrence of leukopenia. Thus, it would be advantageous to provide a platelet depletion or collection procedure in which such improved MNC return is possible.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately.

In one aspect, a method (not claimed) is provided for separating platelets from the blood of a blood source. The method includes determining a volume of blood to be processed, and/or a volume of platelets to be collected, and/or a time at which to complete blood draw, and/or a time required to complete blood draw from a source during a blood separation procedure. A procedure setpoint calculated from the completion of blood draw is selected or determined. Blood is then drawn from the source and conveyed to a separator. At least a portion of the platelet-containing fraction is conveyed from the separator while increasing a volume of the mononuclear cell-containing fraction in the separator. The mononuclear cell-containing fraction is conveyed from the separator to the source at the procedure setpoint. The blood draw and blood separation is then ended.

In another aspect, a blood separation device is provided for use in combination with a fluid flow circuit. A blood separation device according to the present invention comprises the technical features as defined in independent claim 1. The blood separation device includes a separator a pump system, and a controller. The controller is configured to (a) determine a volume of blood to be processed, and/or a volume of platelets to be collected, and/or a time at which to complete blood draw, and/or a time required to complete blood draw from a source during a blood separation procedure, (b) select or determine a procedure setpoint calculated from the completion of the blood draw, (c) execute a blood separation procedure and (d) end blood draw. The blood separation procedure includes operating the pump system to draw blood from a source into the separator, operating the separator to separate the blood into a platelet-containing fraction and a mononuclear cell-containing fraction, operating the pump system to convey at least a portion of the platelet-containing fraction from the separator while increasing a volume of the mononuclear cell-containing fraction in the separator, and operating the pump system to convey the mononuclear cell-containing fraction out of the separator to the source at the procedure setpoint.

### Brief Description of the Drawings

Fig. 1 is a is a perspective view of an exemplary commercial blood separation device;
Fig. 2 is a diagrammatic view of an exemplary disposable fluid flow circuit that may be used in combination with the blood separation device of Fig. 1;
Fig. 3 is a side elevation view, with portions broken away and in section, of the blood separation device of Fig. 1, with a centrifuge bowl and spool of the device being shown in their operating position and with the fluid flow circuit of Fig. 2 mounted thereon;
Fig. 4 is a top perspective view of the spool of the centrifuge shown in Fig. 3 in its upright position and carrying the blood separation chamber of the fluid flow circuit of Fig. 2;
Fig. 5 is a plan view of the blood separation chamber shown in Fig. 4, out of association with the spool;
Fig. 6 is an enlarged perspective view of an interface ramp carried by the centrifuge in association with the blood separation chamber, showing the centrifugally separated red blood cell layer and platelet-rich plasma layer within the chamber when in a desired location on the ramp;
Fig. 7 is an enlarged perspective view of the position of platelets/platelet concentrate and platelet-poor plasma in a second stage of the blood separation chamber during a platelet depletion or collection procedure;

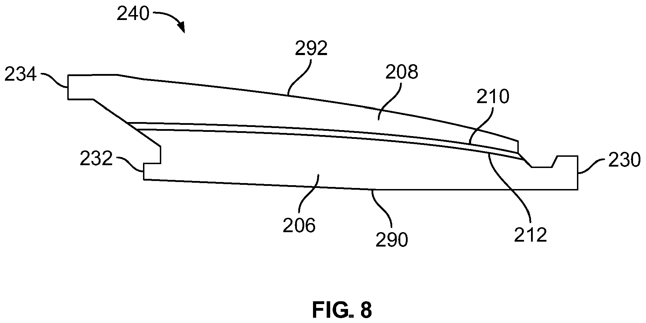

Fig. 8 is a schematic view of the first stage of the blood separation chamber during a portion of a procedure according to the present disclosure; and

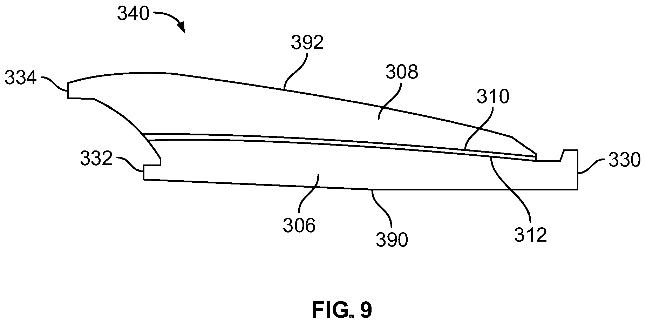

Fig. 9 is a schematic view of the first stage of the blood separation chamber during a second portion of a procedure according to the present disclosure.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

Platelet collection and depletion procedures according to the current disclosure may proceed similarly to the above-described procedure and be executed using a system similar to the one shown in Figs. 1-7. However, it should be understood that the principles described herein are not limited to a particularly configured system and/or a particular sequence of steps or stages. Rather, the MNC-return principles described herein may be applied using a variety of differently configured blood processing systems (e.g., the above-described AMICUS^{®} and TRIMA ACCEL^{®} systems) that carry out platelet collection and/or depletion procedures in different ways. Indeed, it is contemplated that the principles described herein may be applied to any blood processing system and platelet collection or depletion procedure in which MNCs (e.g., in a buffy coat) are allowed to accumulate in a separator (configured as a centrifuge or otherwise configured) over the course of a procedure.

Platelet collection and depletion procedures according to the present disclosure are able to return more MNCs to a donor or patient (leaving only on the order of 1 x 10⁷ MNCs in a separation chamber when using an AMICUS^{®}-type system, for example) by establishing a procedure setpoint calculated from the end of blood draw and executing an MNC-return step at that time. The end of blood draw may be determined according to any suitable approach. This may include the use of one or more pre-counts, such as a platelet pre-count, which is indicative of the platelet content of the blood of a patient or donor before a procedure. Other relevant information may include, for example, a target volume or amount of platelets to be harvested and/or a target platelet post-count (which is indicative of the platelet content of the blood of a patient or donor at the end of a procedure). While it may be advantageous to have information regarding the composition of the blood of a patient or donor prior to beginning a procedure, it should be understood that it is not required. For example, if a platelet pre-count is unavailable, the platelet content of the blood may be determined during the course of a procedure using any suitable approach.

With the appropriate information, along with knowledge of the separation efficiency of the separation device, it is possible to determine how much blood must be drawn from the patient or donor in order to harvest a target volume or amount of platelets or to achieve a target platelet post-count. A system controller may use that calculated volume of drawn blood to determine (based on the expected operational rates of the various components of the system) the time at which blood draw will end (when the starting time is known or when a patient or donor is only available until a particular time of day) and/or the duration of blood draw. Thus, it should be understood that the end point of blood draw may be expressed in a variety of ways - the volume of blood to be drawn, the time at which the proper volume of blood will have been drawn, the amount of time required to draw the proper volume of blood, etc. The controller may be configured to adjust its calculation of the end of blood draw during the course of a procedure, as necessary, such as if there are any unexpected delays (e.g., if blood draw is slower than expected due to a blockage or other flow irregularity).

The system controller may then establish a procedure setpoint that is calculated from the expected end of blood draw. Just as the end of blood draw may be expressed in a variety of ways, the procedure setpoint may be expressed in a variety of ways - a volume of blood, a time of day, an amount of time, etc. For example, when the end of blood draw is expressed in terms of the volume of blood to be drawn, the controller may select or be provided with a specific volume from the end of blood draw, which becomes the procedure setpoint at which point MNCs are returned to the blood source. If a volume is selected as a basis for establishing a procedure setpoint, the specific volume may vary without departing from the scope of the present disclosure (depending on factors such as the configuration of the separation chamber), though it may be advantageous to select a volume that is sufficiently large to ensure that all or at least substantially all of the MNCs in the separation chamber will be displaced from the separation chamber by the chosen volume of blood flowing into the chamber. In an exemplary embodiment in which 2 liters of blood are to be drawn from the donor or patient, a volume in the range of about 25 mL to about 100 mL may be selected. If a volume of 25 mL is selected, the procedure setpoint will be set to be when 1.975 liters of blood have been drawn from the donor. In another example, when the end of blood draw is expressed in terms of a time at which blood draw must end (e.g., when a donor or patient is only available until a certain time of day), the controller may select or be provided with a specific time from the end of blood draw at which to return MNCs in the separation chamber to the blood source.

If a time is selected as a basis for establishing a procedure setpoint, the specific volume may vary without departing from the scope of the present disclosure (depending on factors such as the configuration of the separation chamber), though it may be advantageous to select a time that is sufficiently long to ensure that all or at least substantially all of the MNCs in the separation chamber will be displaced from the separation chamber by the blood flowing into the chamber during that time. The specific time may be from about one minute to about five minutes from the end of the procedure, for example. This may include setting the procedure setpoint at a particular time (e.g., at 11:55 when blood draw must be ended at 12:00 and a time of five minutes is selected) or at a certain amount of time before the end of blood draw (e.g., if blood draw is calculated to continue for one hour, the procedure setpoint can be set to two minutes from end of the procedure, or after fifty-eight minutes have passed). If the calculated duration of blood draw changes during the course of the procedure, the time at which MNC return is executed may also be changed. For example, in the preceding example in which blood draw is initially calculated to last for one hour, with a procedure setpoint of two minutes, if there is a delay of five minutes during the course of the procedure, the recalculated duration of blood draw will be one hour and five minutes. In this case, the time from the end of blood draw at which MNC return will remain the same (two minutes before the end of blood draw), but the time at which MNC return will be adjusted (from fifty-eight minutes after blood draw has begun to one hour and three minutes after blood draw has begun).

After setting the procedure setpoint and performing any pre-procedure steps (such as priming the circuit), the system controller instructs one or more of the pumps of the device to draw blood from the blood source (donor/patient) into a separation chamber. The blood enters the separation chamber and the buffy coat or MNC-containing fraction is separated from the other blood components, with some or all of the other components being collected or returned to the source while the volume of the buffy coat or MNC-containing within the separation chamber increases. As noted above, the configuration of the separation chamber and the manner in which the buffy coat or MNC-containing fraction is separated from the other blood components may vary without departing from the scope of the present disclosure.

By way of an exemplary embodiment, during a platelet collection or depletion procedure carried out using the AMICUS^{®} separator, the blood drawn from the source enters the first stage 240 of the separation chamber illustrated in Fig. 8 via an inlet port 232 and is separated into red blood cells, or a red blood cell fraction 206 (i.e., the high density blood component), and platelet-rich plasma, or a plasma fraction 208 (i.e., the low density blood component). The red blood cells are removed from the first stage 240 (and typically returned to the blood source) via an outlet port 230, while the platelet-rich plasma is conveyed out of the first stage 240 a second outlet port 234 and into the second stage of the chamber via an inlet port. In the second stage, the platelet-rich plasma is separated into platelet-poor plasma and platelets or platelet concentrate. The platelet-poor plasma is removed from the second stage (and may be returned to the blood source) via an outlet port, leaving the platelets/platelet concentrate to accumulate in the second stage for eventual transfer to a collection container.

As described above, at or adjacent to the interface 210 between the red blood cell fraction 206 and the plasma fraction 208, a buffy coat or MNC-containing fraction 212 will arise, as the MNCs and other blood cells (namely, platelets and leukocytes) are sedimented from the red blood cells. For the bulk of the procedure, the MNC-containing fraction 212 resides largely at the interface 210 while the plasma fraction 208 exits from the first stage 240 to the second stage and the red blood cell fraction 206 exits to be returned to the donor/patient. The interface 210 is kept at a first position (illustrated in Fig. 8) that is a particular distance between the high-G wall 290 and the low-G wall 292 during this time. This position may be expressed as the percentage of the thickness or height of the first stage 240 that is occupied by the red blood cell fraction 206, as presented on an interface ramp associated with the first stage 240. In one embodiment, this can be from 30-50% (i.e., with the red blood cell fraction 206 occupying 30-50% of the space between the high-G wall 290 and the low-G wall 292), and in a further embodiment it can be about 40%, for example.

The position of the interface 210 is regulated by the relative rates at which the red blood cell fraction 206 and the plasma fraction 208 are conveyed from the first stage 240. In one embodiment, a pump is associated with the plasma outlet 234, while there is no pump associated with the red blood cell outlet 230, with the flow of red blood cells from the first stage 240 being equal to the difference in the rate at which blood flows into the first stage 240 and the rate at which the plasma fraction 208 is pumped out of the first stage 240. Thus, the position of the interface 210 (and, hence the MNC-containing fraction 212) may be adjusted by changing the rate of operation of the plasma pump, with an increased rate causing the interface 210 to move closer to the low-G wall 292 and a decreased rate causing the interface 210 to move closer to the high-G wall 290. It should be understood that this is merely one possible approach to adjusting and controlling the position of the interface 210 and that other approaches may be employed without departing from the scope of the present disclosure.

Fig. 9 illustrates the positions of the various separated blood components within the first stage during MNC-return at a procedure setpoint. At the procedure setpoint, as described above, the system controller controls the other components of the processing device (e.g., a plasma pump) to cause the interface 310 to move from the first position (shown in Fig. 8) to a second position closer to the high-G wall 390 (Fig. 9). The exact position of the interface 310 at this time may vary without departing from the scope of the present disclosure, with the second position being whatever is appropriate to bring the MNC-containing fraction 312 close enough to the high-G wall 390 so as to exit the first stage 340 via the red blood cell outlet 330. The MNC-containing fraction 312 flows out of the first stage 340 with the red blood cell fraction 306 via the red blood cell outlet 330, with the MNC-containing fraction 312 and the red blood cell fraction 306 being returned to the donor/patient (before blood draw is complete). In an exemplary embodiment (in which the interface is maintained at a 30-50% first position within the first stage during steady state separation), the controller may move the interface to a 20-40% second position. In another exemplary embodiment in which the interface is maintained at a 40% first position, the controller may move the interface to a 30% second position.

As noted above, the position of the interface may be lowered by any acceptable method, such as slowing the pump at the plasma outlet of the first stage. It will be seen that, when the operational rate of a plasma pump is decreased, the volumetric flow rate of the plasma fraction into the second stage of the chamber will be decreased. As such, it may be advantageous to maintain the interface in its second position for only as long as necessary to clear the MNC-containing fraction from the separation chamber so as to not unduly decrease platelet collection efficiency. Thus, depending on what is required to clear the MNC-containing fraction from the separation chamber, it may be necessary for the MNC-return stage to last until the end of blood draw (e.g., beginning two minutes before the end of blood draw and lasting two minutes) or it may instead be acceptable for the MNC-return stage to be ended some time before blood draw ends (e.g., beginning two minutes before the end of blood draw and lasting only one minute before returning to steady state separation conditions). Generally speaking, though, the MNC-return stage executed at the procedure setpoint will typically be relatively brief compared to the duration of blood draw and separation (e.g., one or two minutes for an hour-long procedure), such that the decrease in platelet collection efficiency will be nominal.

Blood draw and separation continue until they are ended by the system controller. The platelets/platelet concentrate accumulating in the second stage are then harvested or collected (e.g., by stopping rotation of the centrifuge and pumping the platelets out of the separation chamber), followed by an optional reinfusion stage in which the fluid that remains in the chamber (including the MNC-containing fraction) is returned to the blood source (as in does at the end of a conventional platelet collection or depletion procedure using the AMICUS^{®} separator).

As described above, modifying a conventional procedure by performing a mid-processing MNC return allows a much higher number of the MNCs to be returned to the donor or patient, reducing the number of MNCs left in the chamber from on the order of 1 × 10⁸ MNCs (at the end of a conventional platelet collection or depletion procedure) to on the order of 1 × 10⁷ MNCs.

As described above, the MNC-return principles described herein are not limited to use with a particular blood separation system. For example, the TRIMA ACCEL^{®} system employs a leukoreduction or "LRS" chamber into which plasma, platelets, white blood cells, and red blood cells are conveyed. The LRS chamber separates the platelets from the white blood cells (including MNCs) and pushes the platelets out to a collection compartment as a leukoreduced platelet product. The white blood cells (including MNCs) can be conveyed into a separate line or stream which is directed back to the donor/patient. Further details for this system can be found in U.S. Patent No. 7,963,901.

Unlike the AMICUS^{®} system, the TRIMA ACCEL^{®} separator does not execute a reinfusion stage at the end of processing, instead scheduling white blood cells to be returned to the blood source at particular time after the procedure has begun. While this approach may appear to be similar to the approach to MNC-return described herein, the distinction between an MNC-return stage scheduled to take place a certain time before the end of blood draw (per the present disclosure) and an MNC-return stage scheduled to take place a certain time after the beginning of blood draw is significant.

As noted above, for any of a number of reasons, blood draw may actually end at a time that differs from an expected time. In this case, scheduling an MNC-return stage to take place a particular time after the beginning of blood draw (which may be intended to occur shortly before the end of blood draw) may result in a larger than expected gap between MNC return and the end of blood draw, with an unacceptably large volume of MNCs remaining in the separator as waste. By employing the principles described herein and scheduling an MNC-return stage to be executed at a particular procedure setpoint calculated from the end of blood draw, it can be ensured that the MNC-return stage will take place a particular time before the end of blood draw, resulting in increased MNC return to the donor or patient.

It will be understood that the embodiments and examples described above are illustrative of some of the applications of the principles of the present subject matter. For these reasons, the scope hereof is not limited to the above description but is as set forth in the following claims.

## Claims

1. A blood separation device for use in combination with a fluid flow circuit, comprising:
a separator;
a pump system; and
a controller configured to
(a) determine a volume of blood to be processed, and/or a volume of platelets to be collected, and/or a time at which to complete blood draw, and/or a time required to complete blood draw from a source during a blood separation procedure,
(b) select or determine a procedure setpoint calculated from the completion of the blood draw,
(c) execute a blood separation procedure comprising
operating the pump system to draw blood from a source into a separator,
operating the separator to separate the blood in the separator into a platelet-containing fraction and a mononuclear cell-containing fraction,
operating the pump system to convey at least a portion of the platelet-containing fraction from the separator while increasing a volume of the mononuclear cell-containing fraction in the separator, and
operating the pump system to convey the mononuclear cell-containing fraction out of the separator to the source at the procedure setpoint, and
(d) end blood draw.

2. The blood separation device of claim 1, wherein the controller is further configured to execute a refusion phase after the end of the blood draw by operating the pump system to convey the contents of the separator to the source.

3. The blood separation device of any one of the preceding claims, wherein the separator includes a first (40; 340) and second (42) stage and the mononuclear cell-containing fraction and the platelet-containing fraction are separated in the first stage within the separator.

4. The blood separation device of any one of the preceding claims, wherein the controller is configured to determine a procedure setpoint that is a set time before the completion of the blood draw.

5. The blood separation device of any one of claims 1-3, wherein the controller is configured to determine a procedure setpoint that is a particular time of day.

6. The blood separation device of any one of claims 1-3, wherein the controller is configured to determine a procedure setpoint that is a set volume of blood remaining to be drawn from the source before the completion of the blood draw.

7. The blood separation device of any one of the preceding claims, wherein the controller is configured to adjust the determination of when blood draw will be completed, with such adjustment changing the time or volume of blood drawn after the beginning of blood draw at which the pump operates to convey the mononuclear cell-containing fraction from the separator to the source at the procedure setpoint occurs without changing the time or volume of blood drawn before the end of blood draw at which the pump operates to convey the mononuclear cell-containing fraction from the separator to the source at the procedure setpoint occurs.

8. The blood separation device of any one of the preceding claims, wherein the controller is configured to execute a separation procedure further comprising
operating the separator to separate the blood into a plasma fraction, a red blood cell fraction, and the mononuclear cell-containing fraction,
operating the pump system to convey the plasma fraction from the first stage within the separator to a second stage within the separator, and
operating the separator to separate the plasma fraction in the second stage into the platelet-containing fraction and a substantially cell-free plasma fraction.

9. The separation device of claim 8, wherein the controller is configured to execute a separation procedure further comprising operating the pump system to decrease the rate at which the plasma fraction is conveyed from the first stage within the separator to the second stage while conveying the mononuclear cell-containing fraction from the separator to the source at the procedure setpoint.

10. The separation device of claim 9, wherein the controller is further configured during a separation procedure to detect a position of an interface (110; 310) between the plasma fraction and the red blood cell fraction in the first stage within the separator, wherein the rate at which the plasma fraction is conveyed from the first stage of the separation stage to the second stage is decreased until the interface moves from a first position within the first stage to a second position within the first stage.

11. The separation device of any one of claims 8-10, wherein the controller is further configured during the separation procedure to maintain the interface at the second position for a predetermined amount of time or volume of blood drawn from the source calculated to convey substantially all of the mononuclear cell-containing fraction from the separator.

12. The separation device of any one of claims 8-11, wherein the operating the pump system to convey the mononuclear cell-containing fraction out of the separator to the source at the procedure setpoint includes operating the pump system to convey the mononuclear cell-containing fraction from the first stage within the separator with the red blood cell fraction.

13. The separation device of any one of the preceding claims, wherein the operating the pump system to convey the mononuclear cell-containing fraction out of the separator to the source at the procedure setpoint lasts until the end of the blood draw.

14. The separation device of any one of claims 1-12, wherein the operating the pump system to convey the mononuclear cell-containing fraction out of the separator to the source at the procedure setpoint does not last until the end of the blood draw.

15. The separation device of any one of the preceding claims, wherein the separator is a centrifuge.

## Patentansprüche

1. Bluttrennvorrichtung zur Verwendung in Kombination mit einem Fluidströmungskreislauf, umfassend:
einen Separator;
ein Pumpsystem; und
eine Steuerung, die konfiguriert ist zum:
(a) Bestimmen eines Volumens an zu behandelndem Blut und/oder eines Volumens an zu sammelnden Blutplättchen und/oder einer Zeit, zu der die Blutentnahme abgeschlossen werden soll, und/oder einer Zeit, die zum Abschließen der Blutentnahme aus einer Quelle während eines Bluttrennvorgangs erforderlich ist,
(b) Auswählen oder Bestimmen eines Verfahrenssollwerts, der aus dem Abschluss der Blutentnahme berechnet wird,
(c) Ausführen eines Bluttrennverfahrens, welches umfasst:
Betreiben des Pumpsystems zum Ziehen von Blut aus einer Quelle in einen Separator,
Betreiben des Separators, um das Blut in dem Separator in eine Fraktion, die Blutplättchen enthält, und eine Fraktion, die mononukleare Zellen enthält, zu trennen,
Betreiben des Pumpsystems, um zumindest einen Teil der Fraktion, die Blutplättchen enthält, aus dem Separator zu fördern, während ein Volumen der Fraktion, die mononukleare Zellen enthält, in dem Separator erhöht wird, und
Betreiben des Pumpsystems, um die Fraktion, die mononukleare Zellen enthält, aus dem Separator zu der Quelle mit dem Verfahrenssollwert zu fördern, und
(d) Beenden der Blutentnahme.

2. Bluttrennvorrichtung nach Anspruch 1, wobei die Steuerung ferner derart konfiguriert ist, dass sie nach Abschluss der Blutentnahme eine Reflow-Phase durchführt, indem sie das Pumpsystem dahingehend betreibt, die Inhalte des Separators zu der Quelle zu fördern.

3. Bluttrennvorrichtung nach einem der vorstehenden Ansprüche, wobei der Separator eine erste (40; 340) und eine zweite (42) Stufe aufweist und die Fraktion, die mononukleare Zellen enthält, und die Fraktion, die Blutplättchen enthält, in der ersten Stufe innerhalb des Separators getrennt werden.

4. Bluttrennvorrichtung nach einem der vorstehenden Ansprüche, wobei die Steuerung derart konfiguriert ist, dass sie einen Verfahrenssollwert bestimmt, bei dem es sich um eine festgelegte Zeit vor dem Abschluss der Blutentnahme handelt.

5. Bluttrennvorrichtung nach einem der Ansprüche 1 bis 3, wobei die Steuerung derart konfiguriert ist, dass sie einen Verfahrenssollwert bestimmt, bei dem es sich um eine bestimmte Tageszeit handelt.

6. Bluttrennvorrichtung nach einem der Ansprüche 1 bis 3, wobei die Steuerung derart konfiguriert ist, dass sie einen Verfahrenssollwert bestimmt, bei dem es sich um ein festgelegtes Volumen an Blut handelt, das vor dem Abschluss der Blutentnahme noch aus der Quelle entnommen werden soll.

7. Bluttrennvorrichtung nach einem der vorstehenden Ansprüche, wobei die Steuerung derart konfiguriert ist, dass sie die Bestimmung, wann die Blutentnahme abgeschlossen sein wird, anpasst, wobei eine solche Anpassung, welche die Zeit oder das Volumen des Blutes verändert, das nach dem Beginn der Blutentnahme entnommen wird, bei der die Pumpe arbeitet, um die Fraktion, die mononukleare Zellen enthält, von dem Separator zu der Quelle mit dem Verfahrenssollwert zu fördern, stattfindet, ohne die Zeit oder das Volumen and Blut zu ändern, das vor dem Ende der Blutentnahme entnommen wird, bei der die Pumpe arbeitet, um die Fraktion, die mononukleare Zellen enthält, von dem Separator zu der Quelle mit dem Verfahrenssollwert zu fördern.

8. Bluttrennvorrichtung nach einem der vorstehenden Ansprüche, wobei die Steuerung derart konfiguriert ist, dass sie einen Trennvorgang ausführt, welcher ferner umfasst:
Betreiben des Separators, um das Blut in eine Plasmafraktion, eine Fraktion aus roten Blutkörperchen und die Fraktion, die mononukleare Zellen enthält, zu trennen,
Betreiben des Pumpsystems, um die Plasmafraktion von der ersten Stufe innerhalb des Separators zu einer zweiten Stufe innerhalb des Separators zu fördern, und
Betreiben des Separators, um die Plasmafraktion in der zweiten Stufe in die Fraktion, die Blutplättchen enthält, und eine im Wesentlichen zellfreie Plasmafraktion zu trennen.

9. Trennvorrichtung nach Anspruch 8, wobei die Steuerung derart konfiguriert ist, dass sie einen Trennvorgang ausführt, der ferner das Betreiben des Pumpsystems umfasst, um die Geschwindigkeit zu verringern, mit der die Plasmafraktion von der ersten Stufe innerhalb des Separators zu der zweiten Stufe gefördert wird, während die Fraktion, die mononukleare Zellen enthält, von dem Separator zu der Quelle mit dem Verfahrenssollwert transportiert wird.

10. Trennvorrichtung nach Anspruch 9, wobei die Steuerung ferner konfiguriert ist, während eines Trennvorgangs eine Position einer Grenzfläche (110; 310) zwischen der Plasmafraktion und der Fraktion aus roten Blutkörperchen in der ersten Stufe innerhalb des Separators zu detektieren, wobei die Geschwindigkeit, mit der die Plasmafraktion von der ersten Stufe der Trennstufe zu der zweiten Stufe gefördert wird, verringert wird, bis sich die Grenzfläche von einer ersten Position innerhalb der ersten Stufe zu einer zweiten Position innerhalb der ersten Stufe bewegt.

11. Trennvorrichtung nach einem der Ansprüche 8 bis 10, wobei die Steuerung ferner derart konfiguriert ist, dass sie während des Trennvorgangs die Grenzfläche für eine vorbestimmte Zeitdauer oder für ein vorbestimmtes Volumen an aus der Quelle entnommenen Blutes, die so berechnet sind, dass im Wesentlichen die gesamte Fraktion, die mononukleare Zellen enthält, aus dem Separator gefördert wird, an der zweiten Position hält.

12. Trennvorrichtung nach einem der Ansprüche 8 bis 11, wobei das Betreiben des Pumpsystems, um die Fraktion, die mononukleare Zellen enthält, aus dem Separator zu der Quelle mit dem Verfahrenssollwert zu fördern, das Betreiben des Pumpsystems umfasst, um die Fraktion, die mononukleare Zellen enthält, von der ersten Stufe innerhalb des Separators mit der Fraktion aus roten Blutkörperchen zu fördern.

13. Trennvorrichtung nach einem der vorstehenden Ansprüche, wobei das Betreiben des Pumpsystems, um die Fraktion, die mononukleare Zellen enthält, von dem Separator zu der Quelle mit dem Verfahrenssollwert zu fördern, bis zum Ende der Blutentnahme andauert.

14. Trennvorrichtung nach einem der Ansprüche 1 bis 12, wobei das Betreiben des Pumpsystems, um die Fraktion, die mononukleare Zellen enthält, von dem Separator zu der Quelle mit dem Verfahrenssollwert zu fördern, nicht bis zum Ende der Blutentnahme andauert.

15. Trennvorrichtung nach einem der vorstehenden Ansprüche, wobei der Separator eine Zentrifuge ist.

## Revendications

1. Dispositif de séparation de sang destiné à être utilisé en combinaison avec un circuit d'écoulement de fluide, comprenant :
un séparateur,
un système de pompage ; et
un contrôleur configuré pour
(a) déterminer un volume de sang devant être traité, et/ou un volume de plaquettes devant être collectées, et/ou un temps auquel le prélèvement sanguin doit être achevé et/ou un temps requis pour achever le prélèvement sanguin depuis une source au cours d'une procédure de séparation de sang,
(b) sélectionner ou déterminer un point de consigne de procédure calculé à partir de l'achèvement du prélèvement de sang,
(c) exécuter une procédure de séparation de sang comprenant
le fonctionnement du système de pompage pour prélever du sang depuis une source dans un séparateur,
le fonctionnement du séparateur pour séparer le sang dans le séparateur en une fraction contenant des plaquettes et en une fraction contenant des cellules mononucléaires,
le fonctionnement du système de pompage pour transporter au moins une partie de la fraction contenant des plaquettes depuis le séparateur tout en augmentant un volume de la fraction contenant des cellules mononucléaires dans le séparateur, et
le fonctionnement du système de pompage pour transporter la fraction contenant des cellules mononucléaires hors du séparateur vers la source au point de consigne de procédure, et
(d) mettre fin au prélèvement de sang.

2. Dispositif de séparation de sang selon la revendication 1, dans lequel le contrôleur est configuré en outre pour exécuter une phase de refusion après la fin du prélèvement de sang en faisant fonctionner le système de pompage pour transporter les contenus du séparateur vers la source.

3. Dispositif de séparation de sang selon l'une quelconque des revendications précédentes, dans lequel le séparateur comporte un premier (40 ; 340) et un deuxième (42) étage et la fraction contenant des cellules mononucléaires et la fraction contenant des plaquettes sont séparées dans le premier étage à l'intérieur du séparateur.

4. Dispositif de séparation de sang selon l'une quelconque des revendications précédentes, dans lequel le contrôleur est configuré pour déterminer un point de consigne de procédure qui est un temps déterminé avant l'achèvement du prélèvement de sang.

5. Dispositif de séparation du sang selon l'une quelconque des revendications 1 à 3, dans lequel le contrôleur est configuré pour déterminer un point de consigne de procédure qui est un moment particulier de la journée.

6. Dispositif de séparation de sang selon l'une quelconque des revendications 1 à 3, dans lequel le contrôleur est configuré pour déterminer un point de consigne de procédure qui est un volume déterminé de sang restant à prélever depuis la source avant l'achèvement du prélèvement de sang.

7. Dispositif de séparation de sang selon l'une quelconque des revendications précédentes, dans lequel le contrôleur est configuré pour ajuster la détermination de savoir le moment auquel le prélèvement de sang sera achevé, ledit ajustement modifiant le temps ou le volume de sang prélevé après le début du prélèvement de sang auquel la pompe fonctionne pour transporter la fraction contenant les cellules mononucléaires depuis le séparateur vers la source au point de consigne de procédure ayant lieu sans modification du temps ou du volume du sang prélève avant la fin du prélèvement de sang auquel la pompe fonctionne pour transporter la fraction contenant des cellules mononucléaires depuis le séparateur vers la source au point de consigne de procédure.

8. Dispositif de séparation de sang selon l'une quelconque des revendications précédentes, dans lequel le contrôleur est configuré pour exécuter une procédure de séparation comprenant en outre
le fonctionnement du séparateur pour séparer le sang en une fraction de plasma, une fraction de globules rouges, et la fraction contenant des cellules mononucléaires,
le fonctionnement du système de pompage pour transporter la fraction de plasma depuis le premier étage dans le séparateur vers un deuxième étage dans le séparateur, et
le fonctionnement du séparateur pour séparer la fraction de plasma dans le deuxième étage en la fraction contenant des plaquettes et une fraction de plasma sensiblement sans cellules.

9. Dispositif de séparation selon la revendication 8, dans lequel le contrôleur est configuré pour exécuter une procédure de séparation comprenant le fonctionnement du système de pompage pour diminuer la vitesse à laquelle la fraction de plasma est transportée depuis le premier étage dans le séparateur vers le deuxième étage tout en transportant la fraction contenant des cellules mononucléaires depuis le séparateur vers la source au point de consigne de procédure.

10. Dispositif de séparation selon la revendication 9, dans lequel le contrôleur est en outre configuré au cours d'une procédure de séparation pour détecter une position d'une interface (110 ; 310) entre la fraction de plasma et la fraction de globules rouges dans le premier étage à l'intérieur du séparateur, dans lequel la vitesse à laquelle la fraction de plasma est transportée depuis le premier étage de l'étage de séparation vers le deuxième étage est diminuée jusqu'à ce que l'interface se déplace depuis une première position dans le premier étage à une deuxième position dans le premier étage.

11. Dispositif de séparation selon l'une quelconque des revendications 8 à 10, dans lequel le contrôleur est en outre configuré au cours de la procédure de séparation pour maintenir l'interface dans la deuxième position pendant une durée prédéterminée ou un volume prédéterminé de sang prélevé depuis la source calculée/calculé pour transporter sensiblement la totalité de la fraction contenant des cellules mononucléaires depuis le séparateur.

12. Dispositif de séparation selon l'une quelconque des revendications 8 à 11, dans lequel le fonctionnement du système de pompage pour transporter la fraction contenant des cellules mononucléaires hors du séparateur vers la source au point de consigne de procédure comporte le fonctionnement du système de pompage pour transporter la fraction contenant des cellules mononucléaires depuis le premier étage à l'intérieur du séparateur avec la fraction de globules rouges.

13. Dispositif de séparation selon l'une quelconque des revendications précédentes, dans lequel le fonctionnement du système de pompage pour transporter la fraction contenant des cellules mononucléaires hors du séparateur vers la source au point de consigne de procédure dure jusqu'à la fin du prélèvement de sang.

14. Dispositif de séparation selon l'une quelconque des revendications 1 à 12, dans lequel le fonctionnement du système de pompage pour transporter la fraction contenant des cellules mononucléaires hors du séparateur vers la source au point de consigne de procédure ne dure pas jusqu'à la fin du prélèvement de sang.

15. Dispositif de séparation selon l'une quelconque des revendications précédentes, dans lequel le séparateur est une centrifugeuse.
